# EUROPEAN PATENT APPLICATION

(11) **EP 2 769 701 A1**
(43) Date of publication of application: **27.08.2014**
(21) Application number: 11874217.0
(22) Date of filing: 16.12.2011
(51) Int. Cl.: A61F 2/82, A61F 2/86, A61F 2/92, A61F 2/06

(54) **INTRAVASCULAR IMPLANT DEVICE KIT AND PREPARATION METHOD FOR IMPLANT DEVICE**

(30) Priority: 19.10.2011 CN 201110318052
(71) Applicant: Shanghai MicroPort Medical (Group) Co., Ltd., Shanghai 201203 (CN)
(72) Inventor: XIE, Zhiyong, Shanghai 201203 (CN); KANG, Yahong, Shanghai 201203 (CN); JIN, Qiaorong, Shanghai 201203 (CN); CHEN, Bing, Shanghai 201203 (CN); ZHANG, Jiuli, Shanghai 201203 (CN); LV, Jian, Shanghai 201203 (CN); XU, Xiaohong, Shanghai 201203 (CN); WANG, Sen, Shanghai 201203 (CN); LUO, Qiyi, Shanghai 201203 (CN)
(74) Representative: FDST Patentanwälte
(86) International application number: PCT/CN2011/084143
(87) International publication number: WO 2013/056497

(57) **Abstract**

An intravascular implant device kit and a preparation method for implant device. The implant device is an intravascular stent locally covered with a film, which can be implanted in a blood vessel to treat vascular diseases such as aneurysm, particularly intracranial aneurysm. The implant device kit comprises a film material to be coated and a mesh stent, wherein the main component of the film material is alkyl cyanoacrylate. The implant device is directly filmed on site at the position of the aneurysm neck by the preparation method, which enables the aneurysm neck to be accurately and quickly blocked, the branch to be kept patent, and the space occupying effect to be obviously improved, and thus realizing a real cure of the aneurysm.

## Description

### Technical Field

The present invention relates to medical instrument field. More particularly, the present invention relates to an intravascular implant device kit and a preparation method for implant device. This device can be implanted into blood vessel to treat vascular malformation diseases such as aneurysm, especially intracranial aneurysm.

### Background

Under the impact of various internal and external factors including mechanical damage, arteriosclerosis, hypertension, vascular smooth muscle cells proliferation, bacteria or virus infection, venous valve disease induction, blood flow impact and the like, it is possible that a vascular wall, in particular an arterial wall, will show a local abnormal dilation or bulge, which is called aneurysm. Aneurysms can happen at different body parts and abdominal aortic aneurysm and intracranial aneurysm are the most common. Intracranial aneurysm as a cystic dilatation of the intracranial vascular wall can grow continuously under the impact of blood flow and oppress the surrounding brain tissues to cause a symptom. The greatest danger associated with an intracranial aneurysm, also called "an untimed bomb", is acute hemorrhage caused by the rupture of the aneurysm due to increased blood pressure or other factors. The mortality in each rupture and bleeding event is about one third. Without a targeted treatment, the hemorrhage can occur again in a short period with extremely low possibility for a patient to survive. Therefore, it is extremely important to treat intracranial aneurysm promptly and correctly.

Intracranial arteries lack surrounding support and are characterized by thin vessel walls, circuitous and multi-bended vessels that are highly branched, sensitivity to stimuli and the like. Intracranial aneurysm is often found at a branched, bifurcated or bended site, primarily due to the hemodynamic factors at this site, that is, the impact of the axial blood flow causes damages in the vascular elastic layer, forming a cystic bulge which then grows into an aneurysm.

Methods for treating an aneurysm typically include the two of surgical operation and vascular intervention therapy. Surgical operation is to block an already-formed aneurysm by means of snipping and ligating the parent artery of the aneurysm, and closing the neck of the aneurysm with a clip so as to shield the aneurysm from the impact of the blood flow. However, this method is time consuming and is associated with traumas, high operational risk and high incidence of postoperational complications.

In recent years, intravascular intervention therapy is gradually becoming the primary method for treating aneurysms with the development in the field of vascular imaging. This method can be classified into two kinds according to the implementation manner thereof.

The first kind of intravascular intervention therapy uses embolic materials to embolize the aneurysm. Typically, with the use of a variety of embolic materials, the embolic agent can be delivered to the cavity of an aneurysm by a microcatheter to embolize the aneurysm so as to achieve the treating effect. Useful embolic materials comprise detachable coils, micro-wire loop, detachable balloon and curable liquid such as NBCA, Glubran and Onyx glue etc. However, the use of solid embolic materials such as coils for embolizing an aneurysm often provides lower embolization rate and tends to induce formation of thrombus in a large scale, leading to cerebral infarction. Commercially available liquid embolic materials in the prior art are not ideal tools because they are either causing serious clingy catheter problem in which case a bleeding can easily occur when the catheter is withdrawn, or are using toxic organic solvent. Other disadvantages include complex handling, lengthy surgical operation, and high relapse rate.

The second kind of intravascular intervention therapy is stent implantation. Covered stents have been used in intravascular intervention therapy for intracranial aneurysms. By using a cover film, a closed duct can be formed in the pathological vessel, protecting the aneurysm against the impact of the blood flow and completely insulating the blood flow at the lesion site from normal blood circulation, thereby achieving the purpose of healing the aneurysm. However, because intracranial arteries are highly branched, a corresponding neurological function will be lost if a covered stent covers an important branch of the vessel. It is also reported that dense mesh stent - flow-diverter can be used to treat aneurysm. The dense mesh stent works in such a way that the stent is fully expanded at the lesion site, the mesh of the stent being small enough to alter the hemodynamics at the neck of the aneurysm to allow the formation of thrombus in the aneurysm, and at the same time, the branch vessel of the vessel equipped with the dense mesh stent being left unblocked due to the presence of a difference in liquid pressure. Relative to covered stents, the application of dense mesh stents is extended. However, due to the contradiction between blocking the aneurysm and keeping the branch unblocked, it is difficult to accomplish both. In addition, there is a significant difficulty in remedying a ruptured aneurysm by this method. Clinically, "bi-stent" or "multi-stent" techniques are also employed by some people to get a better blocking effect so as to treat an intracranial aneurysm. Such techniques increase the mesh density at the neck of the aneurysm by overlapping the stents in a staggered manner to relatively reduce the size of the stent mesh at the neck of the aneurysm. However, this method has a risk of blocking the branch vessel. At present, the defects of dense mesh stents are mainly that the design and processing thereof are limited, the mesh size is relatively large and the mesh density can hardly be improved. Furthermore, it is impossible to arrive at a design which has higher mesh density and smaller mesh size only at the position of the aneurysm neck but has lower mesh density and larger mesh size at the position of a branch vessel. There is also a study reporting a preformed stent locally covered with a film, wherein a confined portion of the stent is coated with a film using polyurethane as the base material, for the purpose of preventing blood flowing into the aneurysm by covering up the neck of the aneurysm with this confined area coated with the film. However, it would be difficult to accurately release the compressed stent in a circuitous and complicated vessel at a position that is right at the neck of an aneurysm.

Regarding stents, Jianmin LIU et al. (see CN101991477A) disclose a vascular reconstructive stent. The vascular reconstructive stent is a mesh tubular structure having a plurality of meshes formed by interlacing a plurality of wire chains, wherein each wire chain is continuous, and nodes of the mesh are movable. The vascular reconstructive stent of the invention has high softness and flexibility, can pass through curve and fine brain blood vessels to reach a target lesion, and conforms to the curve blood vessels so as to maintain the blood vessel inner cavity. This stent has high-density mesh, and can obviously change the blood circulation in the aneurysm so that the blood can be detained to form blood clots and organic solids.

Regarding covered stents, Zhenkui SUN et al. (see Journal of Interventional Radiology, Vol. 19(4), 2010) provides preliminary results showing that Willis covered stent is effective in treating CICA aneurysm and has good conformity. Willis covered stent is a non-locally-covered stent. Ionita et al. (see Proc Soc Photo Opt Instrum Eng. 2009 January 1; 7262: 72621H1-72621H10) uses a novel polyurethane asymmetric vascular stent to treat aneurysm in animal model. The stent is a vascular stent partly covered with a polyurethane patch for blocking the aneurysm neck and thereby preventing the blood from flowing into the aneurysm. The results show that the proximal end is completely healed but the distal end is partially closed, and further investigation about the aneurysm treatment would be required. In addition, this locally-covered stent has the defects of inaccurate localization, posing difficulty for the microcatheter to rotate three dimensionally therein, and the like.

Regarding embolic materials, Xia TIAN et al. (CN86107191) discloses a vascular embolic formulation prepared from n-octyl alpha-cyanoacrylate, tantalum powder and iodized oil (myodil) at the ratio of volume percentages of 80-85: 1-5: 10-15, preferably 83.75: 2.5: 13.75, wherein the single component has a good stability with convenience of use; and the preparation method thereof. The formulation is a permanent vascular embolic material which aggregates rapidly *in vivo* accompanying with low exotherm, resulting in a soft aggregate which cannot be absorbed by blood or tissue fluid, or degraded, so as to lose its efficacy, and can be developed *in vivo* under the irradiation of X-ray. Chinese patent application No. 201110172325.4 also discloses a novel collagen-based liquid embolic material, wherein the alkyl cyanoacrylate and the collagen contained therein self-assemble into a multiple-junction polymer during embolization, to thereby produce an embolic material system. In one aspect, the alkyl cyanoacrylate, as a typical liquid embolic material, has a good embolic effect; and, in another aspect, the collagen can absorb water and expand rapidly, and can induce the growth of endothelial growth factor upon embolization, which provides an embolic body with a low density and a good flexibility, as well as a permanent embolization with no recurrence. The disclosure of this application is incorporated herein by reference.

In addition, Kerber et al. (US 2009/0137981 A1) discloses a method for treating a blood vessel. Said method comprises providing at least one manipulable tool in a blood vessel, depositing a non-solid polymerizable material into a deposition area of the vessel, wherein the polymerizable liquid hardens over time upon contact with blood in the blood vessel, and altering the shape of the polymerizable material while it hardens by manipulating the tool.

### Summary of the Invention

In order to solve the above technical problems in the prior art, the present invention provides an implant device kit. This implant device kit useful for preparing a locally covered intravascular stent comprises two components, one of which is a film material for coating and covering that can be delivered by a microcatheter to be within the aneurysm to quickly solidify in blood and meanwhile has certain adhesiveness allowing the adhesion and formation of a dense film at any position of a stent so as to prevent blood from flowing into the aneurysm; and the other one of which is a stent having a sufficient mesh density as the backbone of the locally covering film. The stent can pass through the curve and fine cerebral vessels to arrive at the target lesion and can also conform to the circuitous vessels to maintain the blood vessel inner cavity. The two components coordinate with each other leading to both the precise and rapid blocking of the aneurysm neck, and the maintenance of the patency of a branch vessel. Furthermore, the space occupying effect can be remarkably improved and thereby a real cure of the aneurysm can be achieved.

The present invention provides a method for preparing an implant device locally covered with a film, comprising the following steps: placing in the cavity of an aneurysm a microcatheter for delivering a film material, then implanting a stent to the vascular lesion site through a delivery system, releasing the stent, injecting the film material, and the film material forming a film on the surface of the stent at the position of the aneurysm neck.

The present invention further provides another method for preparing an implant device locally covered with a film, comprising the following steps: delivering a stent to a vascular lesion site, releasing the stent, then delivering a film material into the cavity of the aneurysm by a microcatheter passing through the mesh of the stent, and the film material forming a film on the surface of the stent at the position of the aneurysm neck.

According to the present invention, the major component of the film material is an alkyl cyanoacrylate having the structure as shown in the following formula (1): 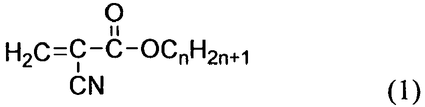 wherein 1≤ n ≤12, preferably 4≤ n ≤10, most preferably 5≤ n ≤8.

This kind of alkyl cyanoacrylates contain cyano and carboxyl groups and have a double electron-withdrawing effect which makes the α-carbon prone to be catalyzed by an environmental anion to quickly polymerize and cure. Blood is rich in anions and thus, it is extremely easy to induce the polymerization of the film material during a surgery to realize on-site film covering.

According to the present invention, in addition to the alkyl cyanoacrylate, said film material further comprises a contrast agent, a polymerization inhibitor and/or a plasticizer. Said polymerization inhibitor which allows stable storage of the film material can be selected from m-methoxyphenol, hydroquinone, phosphoric acid, phosphorus pentoxide, sulfur dioxide, or the like. Said plasticizer which guarantees the softness and flexibility of the film and makes film covering convenient can be selected from dimethyl phthalate, diethyl phthalate, dibutyl phthalate, butyl benzyl phthalate, acetyl tributyl citrate, benzoate, tricresyl phosphate or the like. Contrast agent for angiography is blended in the film material to adapt to the fluorescence X-radiography used during and after the surgery, allowing the film covering details being observable with naked eyes. Said contrast agent comprises iodides such as 6-triiodo formic acid, sodium 6-triiodobenzoate, iothalamic acid, metrizoic acid, iodamide, ioxaglic acid, iopanoic acid, iohexol, iotrolan and the like; bismuth-containing compounds such as bismuth trioxide; barium sulfate; metal powders such as tantalum powder, gold powder and the like; and any mixtures thereof with other common contrast agents. Preferably, said contrast agent is an iodide, gold powder, platinum powder, tantalum powder, titanium powder, tungsten powder or barium sulfate. According to the present invention, the amounts of said polymerization inhibitor, plasticizer and contrast agent to be added are not particularly limited, with preference to the amounts well known in the art.

In a preferred embodiment of the present invention, said film material is blended with a degradable polymer for use in forming the film. Said degradable polymer is selected from one or more of polylactic acid, polyglycolide, polycaprolactone, polyanhydrides, poly(1,2-propylene fumarate), polyphosphazenes, L-tyrosine-derived polymers, polyorthoesters, polymers of amino acids, chitin and derivatives thereof, chitosan and derivatives thereof, hyaluronic acid and derivatives thereof, chondroitin sulfate and derivatives thereof, collagen and derivatives thereof, gelatin and derivatives thereof, agar and derivatives thereof, fibrin and derivatives thereof, and fibroin and derivatives thereof. Preferably, said degradable polymer is collagen or polylactic acid.

According to the present invention, said stent is a mesh tubular stent processed by laser, or a woven stent formed by weaving a continuous wire chain. Preferably, the mesh density of said stent changes asymmetrically along the axial direction.

According to the present invention, the material of said stent is selected from biocompatible metallic materials and/or polymeric materials, or the above degradable polymers.

The implant device of the present invention has the following features:
1) before or after delivering the stent, the film material can be delivered by a microcatheter, a catheter or a specialized delivery measure through circuitous blood vessels and arriving at the target;
2) a precise and rapid blocking can be achieved at the aneurysm neck once the stent is implanted;
3) the stent after being implanted has sufficient conformity to conform to the circuitous cerebral vessels;
4) the stent is only covered with a film at the position of the aneurysm neck but not at other positions, and thus maintains the patency of normal branch arteries.

### Brief Description of Drawings

To illustrate the technical solutions of the present invention more clearly, the present invention will be introduced briefly below with reference to the figures. Obviously, these figures only represent some specific embodiments of the present application. The present invention comprises but is not limited to these figures.

Figure 1 shows a stent with an uneven mesh structure, wherein the part of the stent covering the neck of the aneurysm on the parent artery has higher mesh density (i.e., larger metal coverage rate), and other parts of the stent have lower mesh density (i.e., smaller metal coverage rate).

Figure 2 shows a stent with an uneven mesh structure, wherein part of the neck of the aneurysm is covered with denser mesh and the branch vessel is covered with less dense mesh.

Figure 3 shows a stent with an even mesh structure.

### Detail Description of the Invention

For a further understanding of the present invention, preferred embodiments of the present invention will be described in conjunction with the following Examples. Such description is only to exemplify the features and advantages of the implant device of the present invention and the preparation method thereof, but not to limit the scope of the present invention.

In the cavity of an aneurysm is placed a microcatheter for delivering a film material, followed by implanting a stent to the aneurysm lesion site through the delivery system, releasing the stent, and injecting the film material. The film material cures and adheres to the stent forming a dense film at the position of the aneurysm neck; thereby the stent is covered locally with the film and is accurately located. Alternatively, if the stent is a mesh tubular stent, the microcatheter for delivering the film material can also arrive at the cavity of the aneurysm by passing through the mesh of the stent after the stent is released, then the film can be formed at the neck of the aneurysm.

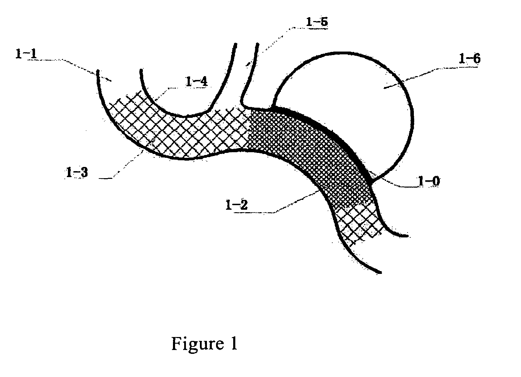

The implant device of the present invention is consisted of a stent and a film (1-0) delivered with a microcatheter and being coated at the scene of the surgery (see Figure 1). Said film (1-0) is formed via polymerization and curing of the film material comprising n-octyl cyanoacrylate and tantalum powder. Said film (1-0) can further be formed via polymerization and curing of a mixture of collagen with the film material comprising n-octyl cyanoacrylate and tantalum powder. Detailed procedures are as follows: collagen particles having a particle size of about 50mesh are produced by granulating 0.5mol of collagen on a fluidized bed (see the article "Study on Optimal Preparation of Protein Powder with Lecithin Using Spray Granulation" by Jiang Zhao et al., published on Food Research and Development, 2007); then the collagen particles are added into 3mol of n-octyl cyanoacrylate monomers and are ultrasonically dispersed homogenously in the monomers; 30g of tantalum nanopowders are added at 37-50°C in an argon atmosphere with stirring to produce a evenly dispersed magnetic fluid of n-octyl cyanoacrylate.

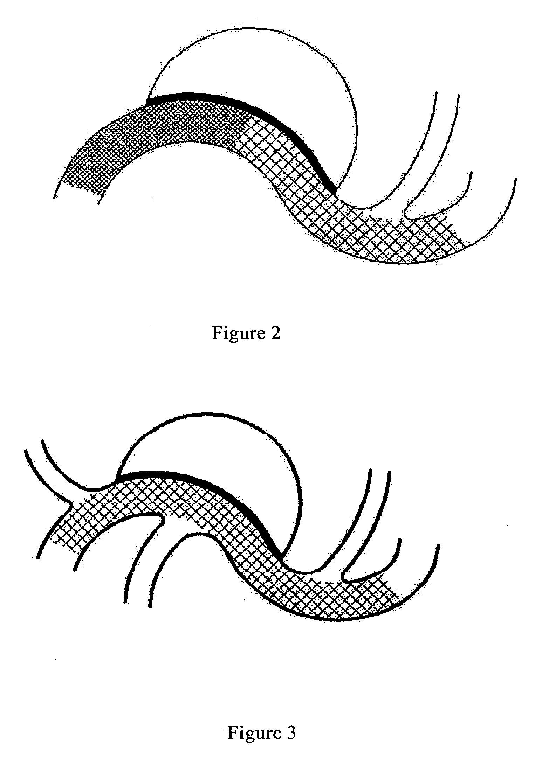

The stent can be one with an uneven mesh structure, i.e., having different metal coverage rates at different positions. The portion of the stent with a higher mesh density (1-2) covers the surface on the neck of the aneurysm (1-6) on the parent artery (1-1), to alter the blood flow in the aneurysm to a maximum extent. Other portions of the stent are structures having lower mesh density (1-3) which provide support for the stent to conform to the normal vascular wall (1-4) so as to maintain the patency of the lumen and reduces as much as possible the covering of the branch vessel (1-5). For a stent with an uneven mesh structure, when part of the neck of the aneurysm is covered with denser mesh and the branch vessel is covered with less dense mesh, the present invention can also achieve the treatment of the aneurysm (see Figure 2). Alternatively, a stent with an even mesh structure can also be used as long as the blood flow in the branch vessel is kept patent, then similarly, the treatment of the aneurysm can totally be accomplished (see Figure 3).

The above device can accurately and rapidly block the aneurysm neck, protecting the aneurysm against the impact of the blood flow. Meanwhile, the blood flow in a normal artery branch can be maintained, remarkably improving the space occupying effect and thereby a real cure of the aneurysm is achieved.

The above description of the examples is merely used to help understand the central concept of the present invention. It should be noted that several improvements and modifications of the device of the present invention could be made by one person of ordinary skill in the art without departing from the principle of the present invention. These improvements and modifications also fall within the scope of the present invention set forth in the claims.

## Claims

1. An intravascular implant device kit comprising a film material to be coated and a mesh stent.

2. The implant device kit according to claim 1, wherein the major component of the film material is an alkyl cyanoacrylate having the structure as shown in the following formula (1): 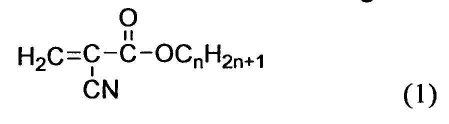 wherein 1≤ n ≤12, preferably 4≤ n ≤10, most preferably 5≤ n ≤8.

3. The implant device kit according to claim 2, wherein the film material further comprises a polymerization inhibitor, a plasticizer and/or a contrast agent, in addition to the alkyl cyanoacrylate.

4. The implant device kit according to any one of the preceding claims, wherein the film material is blended with a degradable polymer for use in forming the film.

5. The implant device kit according to claim 4, wherein the degradable polymer is selected from one or more of polylactic acid, polyglycolide, polycaprolactone, polyanhydrides, poly(1,2-propylene fumarate), polyphosphazenes, L-tyrosine-derived polymers, polyorthoesters, polymers of amino acids, chitin and derivatives thereof, chitosan and derivatives thereof, hyaluronic acid and derivatives thereof, chondroitin sulfate and derivatives thereof, collagen and derivatives thereof, gelatin and derivatives thereof, agar and derivatives thereof, fibrin and derivatives thereof, and fibroin and derivatives thereof.

6. The implant device kit according to any one of the preceding claims, wherein the stent is a mesh tubular stent processed by laser, or a woven stent formed by weaving a continuous wire chain.

7. The implant device kit according to any one of the preceding claims, wherein the material of the stent is selected from biocompatible metallic materials and/or polymeric materials, or the degradable polymers as defined in claim 5.

8. A preparation method for an implant device locally covered with a film, comprising the following steps:
placing in the cavity of an aneurysm a microcatheter for delivering a film material,
then implanting a stent to the vascular lesion site through a delivery system,
releasing the stent,
injecting the film material, and
the film material forming a film on the surface of the stent at the position of the aneurysm neck.

9. A preparation method for an implant device locally covered with a film, comprising the following steps:
delivering a stent to a vascular lesion site,
releasing the stent,
then delivering a film material into the cavity of the aneurysm by a microcatheter passing through the mesh of the stent, and
the film material forming a film on the surface of the stent at the position of the aneurysm neck.

10. The preparation method according to claim 8 or 9, wherein the major component of the film material is an alkyl cyanoacrylate having the structure as shown in the following formula (1): 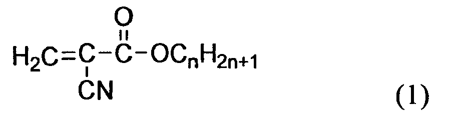 wherein 1≤ n ≤12, preferably 4≤ n ≤10, most preferably 5≤ n ≤8.

11. The preparation method according to claim 10, wherein the film material further comprises a polymerization inhibitor, a plasticizer and/or a contrast agent, in addition to the alkyl cyanoacrylate.

12. The preparation method according to any one of claims 8-11, wherein the film material is blended with a degradable polymer for use in forming the film.

13. The preparation method according to claim 12, wherein the degradable polymer is selected from one or more of polylactic acid, polyglycolide, polycaprolactone, polyanhydrides, poly(1,2-propylene fumarate), polyphosphazenes, L-tyrosine-derived polymers, polyorthoesters, polymers of amino acids, chitin and derivatives thereof, chitosan and derivatives thereof, hyaluronic acid and derivatives thereof, chondroitin sulfate and derivatives thereof, collagen and derivatives thereof, gelatin and derivatives thereof, agar and derivatives thereof, fibrin and derivatives thereof, and fibroin and derivatives thereof.

14. The preparation method according to any one of claims 8-13, wherein the stent is a mesh tubular stent processed by laser, or a woven stent formed by weaving a continuous wire chain.

15. The preparation method according to any one of claims 8-14, wherein the material of the stent is selected from biocompatible metallic materials and/or polymeric materials, or the degradable polymers as defined in claim 13.
